(19) Europäisches Patentamt
European Patent Office
Office européen des brevets

(11) Veröffentlichungsnummer : **0 089 007**
**B1**

(12) # EUROPÄISCHE PATENTSCHRIFT

(45) Veröffentlichungstag der Patentschrift : **30.07.86**

(51) Int. Cl.[4] : **C 12 P 21/02**

(21) Anmeldenummer : **83102337.9**

(22) Anmeldetag : **10.03.83**

(54) **Verfahren zur Umwandlung von Präproinsulinanaloga zu Insulinen.**

(30) Priorität : **13.03.82 DE 3209184**

(43) Veröffentlichungstag der Anmeldung : **21.09.83 Patentblatt 83/38**

(45) Bekanntmachung des Hinweises auf die Patenterteilung : **30.07.86 Patentblatt 86/31**

(84) Benannte Vertragsstaaten : **AT BE CH DE FR GB IT LI LU NL SE**

(56) Entgegenhaltungen :
**EP-A- 0 017 938**
**EP-A- 0 045 187**
**EP-A- 0 056 951**
**CH-A- 599 927**
**US-A- 3 276 961**
**US-A- 3 903 068**
**CHEMICAL ABSTRACTS, Band 95, Nr. 3, 1981, Seite 759, Nr. 25640h, Columbus, Ohio, USA**
**MICROBIOLOGY ABSTRACTS, Band 15, Nr. 10, Oktober 1980, Seite 15, Nr. 9050-A15, IRL, Oxford, G.B. K. MORIHARA et al.: "Achrombacter protease d-catalyzed conversion of porcine insulin into human insulin"**

(73) Patentinhaber : **HOECHST AKTIENGESELLSCHAFT**
**Postfach 80 03 20**
**D-6230 Frankfurt am Main 80 (DE)**

(72) Erfinder : **Obermeier, Rainer, Dr.**
**Langenhainer Weg 14**
**D-6234 Hattersheim am Main (DE)**
Erfinder : **Geiger, Rolf, Prof., Dr.**
**Heinrich-Bleicher-Strasse 33**
**D-6000 Frankfurt am Main 50 (DE)**
Erfinder : **Grau, Ulrich, Dr.**
**Zeil 17**
**D-6238 Hofheim am Taunus (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 089 007 B1**

Jouve, 18, rue St-Denis, 75001 Paris, France

**Beschreibung**

Die Erfindung betrifft ein Verfahren zur Herstellung von Insulinen aus Analoga von Präproinsulinen.

Aus der nicht vorveröffentlichten europäischen Patentanmeldung 56 951 ist ein Verfahren zur Herstellung von Humaninsulin oder seinen Derivaten aus Schweineinsulin oder dessen Derivate bekannt, bei dem man Schweineinsulin bzw. ein Derivat des Schweineinsulins bei einem pH-Wert unterhalb seines isoelektrischen Punktes in Gegenwart von Trypsin oder einem trypsinähnlichen Ferment mit einem Überschuß eines Threoninesters oder eines seiner Derivate umsetzt und gewünschtenfalls danach die Estergruppe abspaltet. Als Derivate des Schweineinsulins werden solche definiert, die « durch Einbau von Schutzgruppen an freiliegenden Funktionen oder durch Abspaltung oder Austausch einzelner Aminosäuren erhalten werden können ». Präproinsuline und deren Analoga unterscheiden sich von den Insulinen u.a. dadurch, daß sie einkettige Proteine darstellen, die durch Disulfidbrücken quervernetzt sind, während das Insulin die Struktur eines zweikettigen Proteins hat.

Mit den verschiedenen Verfahren kann man durch an sich bekannte enzymkatalysierte Transamidierung Schweineinsulin umwandeln (s. z. B. DE-OS 3 104 949). Daneben sind diese einstufigen Verfahren auch geeignet, aus Proinsulin und Intermediat-Insulinen Insulin zu erzeugen. Mit Trypsin findet bei der Schweineinsulin-Umwandlung unter bestimmten verfahrensgemäßen Bedingungen die Transamidierung von Lys$^{B29}$-Ala$^{B30}$OH in -Lys$^{B29}$-Thr-O-Ester statt, aus dem nach bekannten Methoden der Esterpalstung freies Insulin hergestellt wird. Im Falle des Proinsulins oder der Intermediat-Insuline findet die Umamidierung von -Lys$^{B29}$-Ala$^{B30}$-Arg-Arg- zu Lys$^{B29}$-Thr-O-Ester und darüber hinaus an -Lys-Arg$^{AO}$-Gly$^{A1}$-statt.

Das im menschlichen oder tierischen Körper gebildete Insulin fällt zunächst in der Form eines linearen Präproinsulins an, wobei die an das N-terminale Phe$^{B1}$-geknüpfte Präsequenz aus etwa 24 Aminosäure-Resten aufgebaut ist. Als Funktion wird diesem Molekülteil eine Signalwirkung sowohl bei der Proteinbiosynthese als auch beim Transportmechanismus durch die Membran der synthetisierenden Zelle zugeschrieben.

In zunehmendem Maße wird die oben genannte semisynthetische Herstellung von Humaninsulin aus natürlichem Schweineinsulin durch die Gewinnung von Humaninsulin als chimäres Genprodukt modifizierter E. coli DNA verdrängt.

Diese gentechnologischen Methoden erlauben es, durch Variation des Signalnukleotidteils ein dem natürlichen Präproinsulin analoges Produkt zu produzieren. Ziel solcher Aminosäuresequenzänderungen ist die Biosynthese eines analogen Präproinsulins, welches die optimale Spaltung zu Insulin ermöglicht. Bei einem bekannten Verfahren (Riggs A.D., et al. Peptides 1979, ed. E Gross und J. Meienhofer, Pierce Chemical Company, Rockford. Illinois, Seite 985-992) ist die Präsequenz so modifiziert, daß sie über einen Methionin-Rest an das N-terminale Phe$^{B1}$ des Proinsulins geknüpft ist, so daß die -Met-Phe$^{B1}$-Bindung mit Hilfe der BrCN-Methode (Gross E., Witkop B., J. Biol. Chem. 1856 (1962)) gespalten werden kann. Das resultierende Proinsulin wird danach auf enzymatischem Wege (Kemmler A., J. Biol. Chem. *246,* 3786 (1971)) mit Trypsin und Carboxypeptidase B zu Humaninsulin umgewandelt.

Sowohl die BrCN-Spaltung als auch die in zweiter Stufe folgende Behandlung mit dem Enzymgemisch können durch unspezifische Reaktionen beträchtliche Verluste am Endprodukt verursachen. Daher ist es wünschenswert, Verfahren zu entwickeln, aus gentechnologisch gewonnenen analogen Präproinsulinen in hohen Ausbeuten und mit möglichst wenigen Reaktionsschritten Insulin zu gewinnen.

Diese Aufgabe wird durch ein Verfahren zur Herstellung von Insulin aus Analoga von Präproinsulinen gelöst, das dadurch gekennzeichnet ist, daß man Präproinsulinanaloga bei einem pH-Wert unterhalb des isoelektrischen Punktes der zugehörigen Insuline mit Hilfe von Trypsin oder einer trypsinähnlichen Endopeptidase mit einem Ester natürlicher Aminosäuren oder solchen Derivaten davon umsetzt, die Schutzgruppen enthalten, und danach die Erstergruppe und die gegebenenfalls vorhandenen weiteren Schutzgruppen abspaltet. Dabei tritt sowohl eine Transamidierung an den N-terminalen Peptidbindungen von Phe$^{B1}$ und Gly$^{A1}$ als auch am C-Terminus von Lys$^{B29}$ ein.

Analoge Präproinsuline sind solche, die am N-Terminus des Proinsulins Lysin oder Arginin oder deren Acylaminoacyl-Reste als Carboxylende einer Prä-Sequenz tragen (siehe Formel I),

(I)

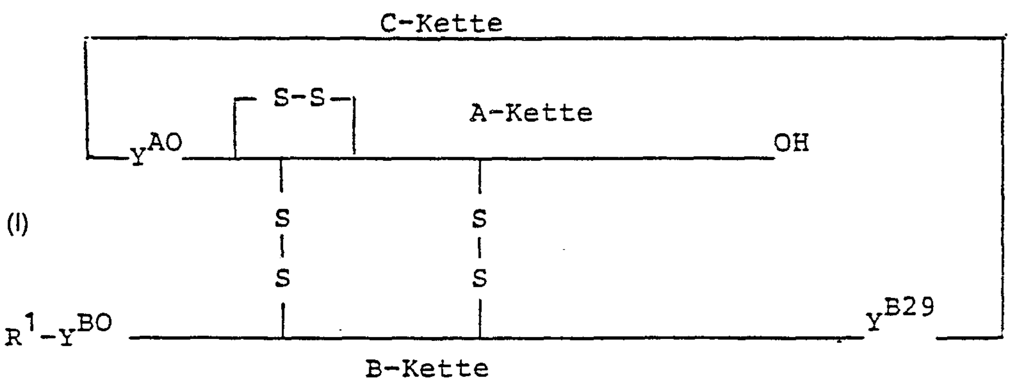

in der Y Lys oder Arg und $R^1$ Wasserstoff, eine L-Aminosäure oder den nachfolgend beschriebenen Peptidrest X bedeuten).

X kann jedes als Signalsequenz dienende Peptid sein. Als Proinsulin-Teil eignet sich jedes Produkt, das mit einem natürlichen oder synthetisch nach bekannten Nukleotid-Darstellungsverfahren variierten Proinsulin codiert wird. Analoge Präproinsuline mit verkürztem C-Peptid-Segment, wie es z. B. beim C-Peptid vom Rind gegenüber dem C-Peptid vom Schwein der Fall ist, kommen ebenfalls für das Verfahren in Frage. Ebenso sind Human-Präproinsulin-analoga geeignet. Wesentlich für die Struktur des C-Peptids ist, daß es über eine basische L-Aminosäure Y = L-Arg oder L-Lys mit dem Glycin der Insulin-A-Kette verknüpft ist ; d. h., daß eine Struktur $Y-(AS)_n-Y$ vorliegt, wobei AS alle codierbaren Aminosäuren bedeutet und n = 0-35 ist. Aminosäureester und/oder deren Derivate mit freier Aminogruppe können für das Verfahren auch so gewahlt werden, daß gewünschtenfalls ein Insulinanalogon von nicht natürlicher Sequenz entsteht.

Bevorzugt sind Ester bzw. Derivate von Estern endständiger Aminosäuren der in Position B 30 natürlich vorkommenden Insuline. Besonders bevorzugt sind die $(C_1-C_6)$-Alkylester (z. B. tert.-Butylester) und die $(C_7-C_{19})$-Aralkylester (z. B. Benzhydryl-, Trityl- oder Fluorenylester), insbesondere Ala-OtBu und Thr(tBu)OtBu. Beispiele geeigneter L-Threoninester sind L-Threonin-tert-butylester, L-Threonin-tert-butyl-O-tert-butylester und L-Threonin-methylester. Unter Derivaten von Treoninestern mit freier Aminogruppe im Sinne der Erfindung sind solche zu verstehen, die an der OH-Funktion des Threonins eine Schutzgruppe, insbesondere die Ether-Schutzgruppe tragen. Besonders bevorzugte Esterschutzgruppen sind $(C_1-C_6)$-Alkyl, insbesondere tert.-Butyl und $(C_7-C_{19})$-Aralkyl, insbesondere Benzhydryl, Trityl oder Fluorenyl.

Der ggf. geschützte Aminosäureester oder sein Derivat mit freier Aminogruppe wird im molaren Verhältnis zum Insulin von 50 bis 500, vorzugsweise von 100 bis 250 eingesetzt. Die Reaktion wird unterhalb eines pH-Wertes von 5,5, bevorzugt zwischen pH 3,8 bis 5,0 bei einer Temperatur zwischen + 2 und + 37 °C, vorzugsweise + 4 und + 10 °C durchgeführt.

Neben Trypsin (s. « The Enzymes », Vol. 4, P.D. Boyer et al., Eds. Acad. Press, N.Y., 2nd Ed. 1960, S. 119-132, ibid. Vol. 3, (3rd Ed. 1971), S. 250-275) eignen sich für das erfindungsgemäße Verfahren auch solche Endopeptidasen, die aus der Literatur als trypsinähnlich bekannt sind, d. h. solche, die spezifisch Peptidbindungen am Carboxylende basischer Aminosäuren spalten (s. z. B. « Kontakte Merck » *1/73* 8-10, *2/73* S. 3-8 ; Z. Physiol. Chem. *348* [1967] 1319 ; Comp. Biochem. Physiol. *28* [1969] 1275 ; Z. Physiol. Chem. *352* [1971] 583 ; Arch. Biochem. Biophys. *129* [1969] 620 ; ibid. *126* [1968] 971 ; Biochem. Biophys. Res. Comm. *37* [1969] 99). Die Menge des anzuwendenden Ferments kann zwischen 1 : 1 und 100 : 1 liegen, wobei sich die Zahlen auf das Gewichtsverhältnis Präproinsulinanalogon : Ferment beziehen. Vorteilhaft arbeitet man bei einem Gewichtsverhältnis von 10 : 1 bis 4 : 1.

Besonders bevorzugte Kombinationen sind Gegenstand der Ansprüche 7 und 8.

Die erfindungsgemäße Umsetzung führt zunächst zu B30-Estern bzw. Schutzgruppen enthaltenden Estern der Insuline, die gewünschtenfalls nach an sich bekannten Verfahren durch Spaltung der Estergruppe und ggf. Abspaltung der Schutzgruppe in das entsprechende freie Insulin übergeführt werden können.

Die Ester können vor ihrer Überführung in die freien Insuline den erforderlichen Reinigungsoperationen, beispielsweise mit Hilfe bekannter säulenchromatographischer Methoden, unterzogen werden.

Das erhaltene Insulin kann in üblichen Darreichungsformen zubereitet und als Arzneimittel zur Behandlung des Diabetes mellitus verwendet werden.

Die in den nachstehenden Beispielen verwendeten Präproinsulinanaloga wurden im wesentlichen nach bekannten Verfahren (Naithani V.K., et al., Insuline, ed. D. Brandenburg, A. Wollmer, 1980, W, de Gruyter, Berlin-New York, Seite 99-106) durch direkte Acylierung von Proinsulin mit den gemischten Anhydriden von BOC-Lys (BOC)OH, BOC-Arg $(AdOC)_2$-OH, BOC-Ala-Gln-Lys (BOC)OH, BOC-Ala-Gln-Arg $(AdOC)_2$-OH, BOC-Gln (tBu)-Pro-Lys (BOC)-Pro-Ala-Arg $(AdOC)_2$-OH und BOC-Gln (tBu)-Pro-Lys (BOC)-Pro-Ala-Lys (BOC)-OH erhalten. Die Reinigung der Verbindungen erfolgte über Ionenaustauscher, die Abspaltung der Schutzgruppen mit Trifluoressigsäure. Die jeweiligen Aminosäureanalysen entsprachen den theoretischen Werten.

## Beispiele

1a) 75 mg $Lys^{B0}$-Proinsulin vom Rind werden zusammen mit 250 mg AlaO (tBu) in 0,5 ml Wasser gelöst und der pH-Wert mit Eisessig auf pH 4,8 eingestellt. Die Lösung wird mit 8 mg Trypsin, gelöst in 0,1 ml Wasser, versetzt. Nach 16 Stunden Stehen bei 4 °C wird die Reaktion durch Fällung mit 68 ml Aceton abgebrochen. Der Niederschlag wird abzentrifugiert. Nach dem Waschen mit Äther wird im Vakuum getrocknet.

Dieses Rohmaterial wird über eine Kieselgelsäule im Laufmittel Chloroform/Methanol/$H_2O$/Triäthylamin/Ameisensäure = 600/500/120/15/3 chromatographiert. Der zuerst eluierte Peak wird gesammelt und im Vakuum auf ein kleines Volumen eingeengt. Die Lösung wird mit 25 ml Aceton versetzt und der präzipitierte Rinderinsulin-B30-ester abzentrifugiert. Der Niederschlag wird mit Äther gewaschen und im Vakuum getrocknet. Ausbeute an Rinderinsulin-B30-tBU : 41 mg.

1b) 25 mg des Rinderinsulins-B30-esters werden in Trifluoressigsäure (0,5 ml) gelöst und bleiben 60 Minuten bei Rautemperatur stehen. Danach wird mit 5 ml Äther versetzt und das ausgefällte freie Rinderinsulin abzentrifugiert und nach Waschen mit Äther im Vakuum getrocknet. Ausbeute : 23 mg.

. Aminosäureanalyse, HPLC-Elutionszeit und biologische Aktivität ergeben mit Rinderinsulin identische Werte.

Vergleichsbeispiel

Um die Tatsache zu belegen, daß nebem der Transamidierung an Lys$^{B29}$ eine Proteolyse zwischen den Positionen $B^O$ und $B^1$ stattfinden kann, wurde folgender Versuch durchgeführt :

500 mg Lys$^{BO}$-Rinderinsulin (Geiger R., Chemiker-Zeitung *100*, 111 (1976) und Geiger, R., Molecular Endocrinology, ed. Mac Intyre & Selke 1977, Elsevier/North Holland Biomedical Press, Seite 27-41) werden in 3,0 ml Wasser zusammen mit 2 g AlaO (tBu) gelöst. Der pH wird mit Essigsäure auf 4,5 eingestellt. Zur Insulin-Lösung werden 20 mg Trypsin gelöst in 0,5 ml Wasser zugefügt. Es wird weiter analog Beispiel 1a verfahren. Die Ausbeuten an Rohmaterial, das laut HPLC-Analyse 67 % Rinderinsulin B30-AlaO (tBu) enthält, beträgt 563 mg. Die Ausbeute an angereichertem Rinderinsulin B30-Ala O(tBu) beträgt nach säulenchromatographischer Reinigung 409 mg.

Die Abspaltung des Esters erfolgt analog Bespiel 1b.

2) 75 mg Ala-Gln-Lys-$^{B30}$-Proinsulin vom Rind (Herstellung nach Naithani et al., l.c.) werden analog Beispiel 1, jedoch bei + 7 °C und pH 4,5 mit Trypsin und AlaOtBu umgesetzt. Nach 16-stündigem Stehen wird aufgearbeitet und der entstandene Rinderinsulin-B30-ester chromatographisch abgetrennt. Die Ausbeute beträgt 47 mg. Die Aminosäureanlayse dieses Materials entspricht dem Rinderinsulin.

Die Abspaltung des Esters erfolgt analog Beispiel 1b.

3) 75 mg Ala-Gln-Lys$^{BO}$-Proinsulin vom Schwein (hergestellt analog Beispiel 2) werden bei + 9 °C und pH 4,4 analog Beispiel 2 mit AlaOtBu umgesetzt und aufgearbeitet. Die Ausbeute an Schweineinsulinester beträgt 39 mg (Aufarbeitung analog Beispiel 1b).

4) 75 mg Ala-Gln-Lys$^{BO}$-Proinsulin vom Schwein werden wie im Beispiel 1a mit 500 mg Thr (tBu) OtBu in Gegenwart von Trypsin umgesetzt. Nach 16 Stunden Reaktionszeit können 49 mg Humaninsulin-B30-(tBu)-OtBu isoliert werden. Es wird analog Beispiel 1b weiterverfahren.

5) 75 mg Arg$^{BO}$-Proinsulin vom Rind werden mit 500 mg Thr (tBu) OtBu wie in Beispiel 1a in Gegenwart von Trypsin umgesetzt. Nach Stehen über Nacht wird aufgearbeitet wie in Beispiel 1a. Die Ausbeute an Rinderinsulin-B30-Thr (tBu) OtBu beträgt 45 mg, das entsprechend Beispiel 1b weiterverarbeitet wird.

6) 75 mg Ala-Gln-Arg$^{BO}$-Proinsulin vom Schwein werden mit 400 mg Thr (tBU) OtBu wie in Beispiel 1a mit 8 mg Trypsin zur Reaktion gebracht. Nach 20 Stunden bei 6 °C beträgt die Ausbeute an Humaninsulin-B30 (tBu) OtBu ; 30 mg. (Weiterverarbeitung analog Beispiel 1b).

7) 5 mg von Gln-Pro-Lys-Pro-Ala-Gln-Lys$^{BO}$-Proinsulin vom Rind werden zusammen mit 15 mg AlaOtBu in 0,1 ml Wasser gelöst. Der pH-Wert wurde mit einer Spur Eisessig auf 4 bis 5 eingestellt und 1 mg Trypsin zugegeben und das Reaktionsgemisch 24 Stunden bei 4 °C gehalten. Mit Hilfe der HPLC werden 63 % Rinderinsulin-B30-tBu gemessen, welches wie im Beispiel 1b weiterbehandelt wird.

8) 5 mg von Gln-Pro-Lys-Pro-Ala-Gln-Arg$^{BO}$-Proinsulin vom Schwein werden mit 25 mg Thr (tBu) OtBu, wie in Beispiel 7 angegeben, zur Reaktion gebracht. Nach 24 Stunden Reaktionszeit werden im HPLC 71 % Humaninsulin-B30 (tBu) OtBu analysiert. Mit Hilfe der präparativen HPLC wird der Ester isoliert. Die Ausbeute beträgt 2,8 mg.

Nach der üblichen Abspaltung der tert.-Butyl-Schutzgruppen ist das Produkt im HPLC und nach Aminosäureanalyse identisch mit Humaninsulin.

9) 75 mg Pro-Ala-Ala-Lys$^{BO}$-Proinsulin vom Schwein werden wie im Beispiel 1a mit 500 mg Thr (tBu) OtBu in Gegenwart von Trypsin umgesetzt. Nach 16 Stunden Reaktionszeit können 52 mg Humaninsulin-B30-(tBu)-OtBu isoliert werden. Es wird analog Beispiel 1b weiterverfahren.

**Patentansprüche**

1. Verfahren zur Herstellung von Insulinen aus Analoga von Präproinsulinen, dadurch gekennzeichnet, daß man Präproinsulinanaloga bei einem pH-Wert unterhalb des isoelektrischen Punktes der zugehörigen Insuline mit Hilfe von Trypsin oder einer trypsinähnlichen Endopeptidase mit einem Ester natürlicher Aminosäuren oder solchen Derivaten daraus, die Schutzgruppen enthalten, umsetzt und danach die Estergruppe und die gegebenenfalls vorhandenen Schutzgruppen abspaltet.

2. Verfahren gemäß Anspruch 1, dadurch gekennzeichnet, daß die verwendeten Aminosäurederivate Ala-OtBu oder Thr (tBu)-OtBu sind.

3. Verfahren gemäß Anspruch 1 oder 2, dadurch gekennzeichnet, daß bei einem pH-Wert zwischen 3,8 und 5,5, vorzugsweise zwischen 3,8 und 5,0 gearbeitet wird.

4. Verfahren gemäß einem oder mehreren der Ansprüche 1 bis 3, dadurch gekennzeichnet, daß das Enzym-Substrat-Verhältnis 1 : 1 bis 1 : 100, vorzugsweise 1 : 4 bis 1 : 10 Gewichsteile beträgt.

5. Verfahren gemäß einem oder mehreren der Ansprüche 1 bis 4, dadurch gekennzeichnet, daß das molare Verhältnis des Aminosäureesters oder seines Schutzgruppen tragenden Derivats zum Präproinsulinanalogen von 50 bis 500, vorzugsweise von 100 bis 250 beträgt.

6. Verfahren gemäß einem oder mehreren der Ansprüche 1 bis 5, dadurch gekennzeichnet, daß die Reaktionstemperatur zwischen + 2 und + 37 °C, vorzugsweise zwischen + 4 und + 10 °C liegt.

7. Verfahren gemäß Anspruch 1 oder 2, dadurch gekennzeichnet, daß bei einem pH-Wert zwischen 3,8 und 5,5 gearbeitet wird, das Enzym-Substrat-Verhältnis 1 : 1 bis 1 : 100 Gewichststeile beträgt, das molare Verhältnis von Aminosäureester oder dessen Derivat zum Präproinsulinanalogen von 50 bis 500 beträgt und die Reaktionstemperatur zwischen + 2 und + 37 °C liegt.

8. Verfahren gemäß Anspruch 1 oder 2, dadurch gekennzeichnet, daß bei einem pH-Wert zwischen 3,8 und 5,0 gearbeitet wird, das Enzym-Substrat-Verhältnis 1 : 4 bis 1 : 10 Gewichtsteile beträgt, das molare Verhältnis von Aminosäureester oder dessen Derivat zum Präproinsulinanalogen von 100 bis 250 beträgt und die Reaktionstemperatur zwischen + 4 und + 10 °C liegt.

## Claims

1. A process for the production of insulins from analogs of preproinsulins, characterized in that preproinsulin analogs are reacted at a pH-value below the isoelectric point of the corresponding insulins in the presence of trypsin or a trypsin-like endopeptidase with an ester of natural amino acids or derivatives of such an ester which contain protective groups, and that the ester group and other protective groups which may optionally be present are then cleaved off.

2. A process as claimed in claim 1, characterized in that the amino acid derivatives used are Ala-OtBu or Thr (tBu) OtBu.

3. A process as claimed in claim 1 or 2, characterized in that it is carried out at a pH-value between 3.8 and 5.5, preferably between 3.8 and 5.0.

4. A process as claimed in one or more of claims 1 to 3, characterized in that the enzyme substrate ratio is from 1 : 1 to 1 : 100 parts by weigths, preferably from 1 : 4 to 1 : 10 parts by weight.

5. A process as claimed in one or more of claims 1 to 4, characterized in that the molar ratio between the amino acid ester or its derivative containing protective groups to the preproinsulin analog is from 50 : 500, preferably from 100 : 250.

6. A process as claimed in one or more of claims 1 to 5, characterized in that the reaction temperature is between + 2 and + 37 °C, preferably between + 4 and + 10 °C.

7. A process as claimed in claim 1 or 2, characterized in that it is carried out at a pH-value between 3.8 and 5.5, the enzyme/substrate ratio is from 1 : 1 to 1 : 100 parts by weight, the molar ratio of amino acid ester or its derivative to the preproinsulin analog is from 50 : 500 and the reaction temperature is between + 2 and + 37 °C.

8. A process as claimed in claim 1 or 2, characterized in that it is carried out at a pH-value between 3.8 and 5.0, the enzyme/substrate ratio is from 1 : 4 to 1 : 10 parts by weight, the molar ratio of amino acid ester or its derivative to the preproinsulin analog is from 100 : 250, and the reaction temperature is between + 4 and + 10 °C.

## Revendications

1. Procédé de préparation d'insulines à partir d'analogues de préproinsulines, caractérisé en ce que l'on fait réagir des analogues de préproinsulines, à un pH inférieur au point isoélectrique des insulines correspondantes, à l'aide de trypsine ou d'une endopeptidase analogue à la trypsine, avec un ester d'amino-acides naturels ou de dérivés de ceux-ci comportant des groupes protecteurs, et en ce qu'ensuite on élimine le groupe ester et les groupes protecteurs éventuellement présents.

2. Procédé selon la revendication 1, caractérisé en ce que les dérivés d'amino-acides employés sont Ala-OtBu ou Thr (tBu) OtBu.

3. Procédé selon la revendication 1 ou 2, caractérisé en ce que l'on opère à un pH compris entre 3,8 et 5,5, de préférence entre 3,8 et 5,0.

4. Procédé selon l'une ou plusieurs des revendications 1 à 3, caractérisé en ce que le rapport enzyme-substrat est de 1/1 à 1/100, de préférence de 1/4 à 1/10 parties en poids.

5. Procédé selon l'une ou plusieurs des revendications 1 à 4, caractérisé en ce que le rapport molaire de l'ester d'amino-acide ou de son dérivé portant des groupes protecteurs aux analogues de préproinsuline va de 50 à 500, de préférence de 100 à 250.

6. Procédé selon l'une ou plusieurs des revendications 1 à 5, caractérisé en ce que la température de réaction est située entre + 2 et + 37 °C, de préférence entre + 4 et + 10 °C.

7. Procédé selon la revendication 1 ou 2, caractérisé en ce que l'on opère à un pH compris entre 3,8 et 5,5, en ce que le rapport enzyme/substrat est de 1/1 à 1/100 parties en poids, en ce que le rapport molaire de l'ester d'amino-acide ou de son dérivé aux analogues de préproinsulines va de 50 à 500 et en ce que la température de réaction est comprise entre + 2 et + 37 °C.

8. Procédé selon la revendication 1 ou 2, caractérisé en ce que l'on opère à un pH compris entre 3,8 et 5,0, en ce que le rapport enzyme/substrat va de 1/4 à 1/10 parties en poids, en ce que le rapport molaire de l'ester d'amino-acide ou de son dérivé aux analogues de préproinsuline va de 100 à 250 et en ce que la température de réaction est comprise entre + 4 et + 10 °C.